# EUROPEAN PATENT SPECIFICATION

(11) **EP 0 421 380 B1**
(45) Date of publication and mention of the grant of the patent: **27.12.1995**
(21) Application number: 90118953.0
(22) Date of filing: 04.10.1990
(51) Int. Cl.: C07K 17/00, C12M 3/00, A61K 35/14, C12N 5/08

(54) **Tumor-lysing cell inducer, method for inducing tumor-lysing cell and device for inducing tumor-lysing cell**
Induzierer einer tumorlysierenden Zelle, Verfahren und Gerät zur Induzierung tumorlysierender Zellen
Inducteur de cellules de lyse de tumeurs, méthode et appareillage pour induire des cellules de lyse de tumeurs

(30) Priority: 03.10.1989 JP 259855/89
(43) Date of publication of application: 10.04.1991
(73) Proprietor: KANEGAFUCHI KAGAKU KOGYO KABUSHIKI KAISHA, Kita-ku Osaka-shi Osaka-fu 530 (JP)
(72) Inventor: Eiji, Ogino, Nada-ku, Kobe-shi, Hyogo-ken (JP); Nobutaka, Tani, Abeno-ku, Osaka-shi, Osaka-fu (JP); Toshio, Hayami, Kakogawa-shi, Hyogo-ken (JP)
(74) Representative: Türk, Gille, Hrabal, Leifert

(56) References cited:
- EP-A- 0 147 689
- WO-A-88/00970
- WO-A-90/04633
- DE-A- 3 634 017
- JOURNAL OF IMMUNOLOGY vol. 143, no. 5, September 1989, Baltimore, USA pages 1461 - 1466; M. KOLBER et al.: "Target cell lysis by cytotoxic T lymphocytes redirected by antibody-coated polystyrene beads. "

## Description

The present invention relates to a tumor-lysing cell inducer which induces cells capable of lysing tumors by the stimulation of leukocytes in a body fluid, and a method for inducing tumor-lysing cells and an inducing device of tumor-lysing cells, which utilize the above-mentioned inducer.

It has been known that in leucocyte fractions, there are cells which have a function for lysing or excluding tumor cells, such as a cytotoxic T lymphocytes, natural killer cells and monocytes. Not only healthy persons but also tumor-bearing patients have these cells in their bodies. Nevertheless, the tumor in the tumor-bearing patient grows, thus his symptom becomes worse.

It has been considered that such a phenomenon is caused by the decrease of the number of the tumor-lysing cells and the lowering of the activity of the tumor-lysing cells due to the immunological tolerance, the activtion of immunosuppressive cells, the shift toward immunosuppressive side in humoral immuno factor balance, and the like.

As one of tratments of malignant tumors, it can be considered that tumor-lysing cells are obtained by stimulating leukocytes. It is very difficult, however, that the tumor-lysing cells are induced by stimulating efficiently and selectively the leukocytes in vivo. Accordingly, a treatment of tumors wherein leukocytes are taken out from a tumor-bearing patient's body, the leukocytes are cultured with stimulating for few days in vitro while adjusting artificially the environment to induce tumor-lysing cells, and the induced tumor-lysing cells are returned into the tumor-bearing patient's body, is tried in various ways.

As stimulants used in the above-mentioned methods, there have been known lymphokines such as interleukin 2 [LAX treatment: E.A. Grimm, "Journal of Experimental Medicine", 155, 1823 (1982)], lectins such as PHA and PWM, protein A, and the like.

Presently, for treatment of malignant tumors, trials wherein lymphocytes are cultured with stimulating in vitro to induce tumor-lysing cells have been lively studied. The treatments have, however, some problems such that
(1) a stimulant used in the induction of the tumor-lysing cells has high toxicity, so there is a great risk if leaking the stimulant;
(2) the tumor-lysing cells are changed in morphology and shape during the few days' culture; and
(3) troublesome operations with the culture are required and a contamination is caused.

In order to solve the problem (1), it is considered that a stimulant having low toxicity is used and the stimulant is fixed in an insoluble carrier to prevent the leakage of stimulant. As to the stimulant having low toxicity, antibodies against leukocytes are laboratorially studied in part. It has been generally known, however, that substances having low toxicity are low in effect for inducing tumor-lysing cells.

Therefore, an effect for inducing tumor-lysing cells by leukocyte antibodies is not very high, for instance, it is only reported to induce tumor-lysing cells having antigenic nonspecificity from tumor-lysing cells having antigenic specificity [J.F.M. Leeuwemberg, "Journal of Immunology", 134 (6), 3770(1985)]. Further, since it is generally known that there are many cases where the bioactivity is lowered by fixing the stimulant, it can assumed that when the leukocyte antibody is fixed, it is not expected to obtain an excellent effect for inducing tumor-lysing cells.

Also, in order to solve the problems (2) and (3), the shortening of the duration of culture or the omission of culture itself is required. It is essential, anyway, to strongly stimulate the leukocytes. Stimulants having strong inducing activity of tumor-lysing cells are, as mentioned above, generally high in toxicity, and even if using the stimulant fixed in an insoluble carrier, it is very difficult to completely prevent the leakage of the stimulant. That is to say, it is almost impossible to put a safe and effective stimulant into a practical use from the view point of safety.

In order to develop a tumor-lysing cell inducer having the safety as well as the high activity for inducing tumor-lysing cells, it is required to obtain simultaneously two properties contradictory to each other as mentioned above, which is very difficult, and, in fact, such an inducer has not yet been realized.

"J. Immunol.", vol. 143, no. 5, pp. 1461-1466 (1989), discloses a very specific method of lysing tumor cells induced by a certain antibody combination. It is described therein that the coexistance of tumor cells, polystyrene beads coated with both of anti-T3 and antitarget cell antibodies, and cytotoxic lymphocytes is significant. That is, an activation must by carried out in the vicinity of target cells.

EP-A-0 147 689 discloses the induction of antitumor immunocytes using insoluble antitumor immunocyte-inducing material constituted of an insoluble carrier and a ligand having T cell lymphocyte-activating properties as for example anti-T cell antibodies and anti-idiotype antibodies.

DE-A-36 34 017 refers to the activation of T cells wherein a solid carrier is used with anti-T cell antibodies fixed theron. However, this process comprises an incubation step or a culturing step which requires complicated operations and strict care for infection.

Therefore, the object of the present invention is to provide a safe and highly effective tumor-lysing cell inducer for treatment of tumors and to provide a method and a device for inducing tumor-lysing cells.

Surprisingly it has been found that even by using a fragment such as F(ab')₂ of an anti-leucocyte antibody the effect of inducing killer activity can be exhibited when the fragment is fixed in the surface of a water-insoluble carrier in a specific amount. This is very surprising in particular in view of the fact that from J.A. Bluestone et al. in "J. Immunol.", 144 (7), 2840-2846 (1990), it has already been known that a fragment F(ab')₂ of anti-CD3 monoclonal antibody (145 2C11) does not exhibit an effect of inducing antitumor activity while the whole anti-CD3 monoclonal antibody exhibits a significant effect.

Thus, the subject-matter of the present invention is a tumor-lysing cell inducer comprising a part of anti-leucocyte antibody, that part being fixed in the surface of a water-insoluble carrier in an amount of from 1 x 10⁻⁹ to 1 x 10⁻² »mol/cm².

According to a preferred embodiment not less than two kinds of parts of anti-leucocyte antibody are fixed in the surface of said water-insoluble carrier.

The carrier used according to the present invention preferably is hydrophilic.

According to a further preferred embodiment of the present invention the anti-leucocyte antibody in the inducer of the present invention is an antibody against at least one surface antigen of the lymphocyte selected from the group consisting of CD2, CD3, CD4, CD8 and CD16.

A further subject-matter of the present invention is a method for inducing tumor-lysing cells which comprises contacting a body fluid containing leukocytes with a tumor-lysing cell inducer, said tumor-lysing cell inducer comprising a part of anti-leukocyte antibody, said part being fixed in the surface of a water-insoluble carrier in an amount of from 1 x 10⁻⁹ »mol/cm² to 1 x 10⁻² »mol/cm².

The important characteristic of the method of the present invention is that effector cells can be activated only by bringing them into contact with a fragment of the antibody fixed in the surface of a water-insoluble carrier without any incubation or culturing step, for example only by flowing suspension of effector cells (or blood) through a device containing the fixed fragment in a column. So, the method of the present invention can be applied even for an extracorporeal circulation because of the short time required for activation of the effector cells.

Finally, a third subject-matter of the present invention is a device for inducing tumor-lysing cells which comprises a tumor-lysing cell inducer comprising a part of antileukocyte antibody, said part being fixed in the surface of a water-insoluble carrier in an amount of from 1 x 10⁻⁹ »mol/cm² to 1 x 10⁻² »mol/cm², and a container having an inlet and an outlet for body fluid and a preventing means of the outpouring of said inducer from the container, said inducer being packed in said container.

The tumor-lysing cell inducer of the present invention is safe and has strong killer activity compared to any stimulants capable of inducing tumor-lysing cells which have hitherto been known.

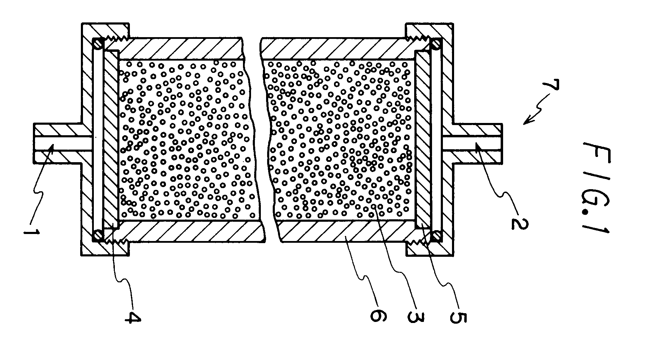

Fig. 1 is a cross sectional view of one embodiment of the devices for inducing tumor-lysing cells of the present invention.

The term "body fluid" used herein means a humoral liquid component derived from a living body, for instance, blood, plasma, serum, ascites, lymph liquid, synovia in articular cavity, and fractions obtained thereform.

The term "anti-leukocyte antibody" used herein means an ntibody prepared by using a leukocyte as an antigen. The leukocytes include e.g. a lymphocyte, a monocyte, a neutrophil, a basophil, and an eosinophil. Among anti-leukocyte antibodies, an antilymphocyte antibody is preferred. The lymphocyte has various surface antigens on its surface. It has been decided that the surface antigens are called by a CD code (cluster differentiation) in an international workshop.

Among the antibodies against the surface antigens of the lymphocyte, antibodies against CD2, CD3, CD4, CD8 and CD16 are particularly preferred. In the selection of an antibody to be fixed from among the anti-leukocyte antibodies, various kinds of antibodies against various kinds of antigens may be used as well as one kind of an antibody against one kind of an antigen may be used. In case of using one kind of the antibody, the antibody against CD2, CD3 oder CD16 is particularly preferable, though it is not limited thereto. Also, in case of using various kinds of the antibodies, mixtures containing the antibody against CD2, CD3 or CD16 are particularly preferable, though various kinds of the mixtures are considered.

As the kinds of the antibody itself, there are e.g. IgG, IgM, IgA, IgD and IgE, and any kinds of antibodies can be used. Also, there are antibodies derived from animals such as a mouse, a rat, a rabbit and a goat and antibodies derived from a human. The antibodies derived from humans are preferable from the viewpoint of the safety including the antigenicity, though there is no particular limitation as to the antibody.

The term "a part of the antibody" used herein means a fragment of the antibody or a plurality of the fragments combined with covalent bond and having a lower molecular weight than the molecular weight of the antibody. The antibody can be severed in any method, for instance, it is served by using an enzyme or a chemical agent, or is genetically engineeringly severed.

The water-insoluble carrier used in the present invention is in the state of a solid at the ordinal temperature under original pressure and is water-insoluble. It is desirable that the carrier has at least one functional group on its surface, for binding the part of the antileukocyte antibody with the carrier.

Examples of the carrier are for instance, an inorganic carrier such as glass beads or a silica gel; an organic carrier such as a synthetic polymer, e.g. crosslinked polyvinyl alcohol, crosslinked polyacrylate, crosslinked polyacrylamide or crosslinked polystyrene, or a polysaccharide e.g. crystalline cellulose, crosslinked cellulose, crosslinked agarose or crosslinked dextran; a complex carrier such as a carrier made of the two kinds of the organic carrier, a carrier made of the organic carrier and the inorganic carrier.

Examples of the functional groups existing on the carrier surface are, for instance, hydroxyl group, amino group, aldehyde group, carboxyl group, thiol group, silanol group, amido group epoxy group, a halogen atom, succinylimide group, and an acid anhydride group.

Also, it is preferable that the water-insoluble carrier is hydrophilic, for preventing the deterioration of the tumor-lysing cells, which is caused by adhering the cells to the surface of the tumor-lysing cell inducer with nonspecifically hydrophobic interaction.

The tumor-lysing cell inducers of the present invention are characterized in that the part of the anti-leukocyte antibody is fixed in the water-insoluble carrier. For obtaining such tumor-lysing cell inducers, the part of the anti-leukocyte antibody can be fixed in the water-insoluble carrier in any way, for instance with covalent bond, hydrophobic bond or ion bond. Among them, it is particularly preferable that the antibody is fixed in the carrier by covalent bond from the viewpoint of the safety during the sterilization or treatment.

In case of fixing the antibody in the water-insoluble carrier with covalent bond, there are various ways and any ways can be applied to the present invention. For instance, when utilizing a functional group exerting on the antibody molecule as it is, the antibody is bonded with the carrier e.g. through an ester (including a thioester) bond, an amido (including a sulfonamido) bond, an ether (including a thioether) bond, amino bond, and imide bond. Also, there is a way wherein after introducing a functional group having high reactivity into the antibody molecule, the antibody is bonded with the water-insoluble carrier. There are various agents used for introducing functional groups having higher reactivity into the antibody, and DSC and WSC are particularly effective as the agent for introducing functional groups.

It is preferable that the tumor-lysing cell inducer of the present invention has a proper amount of the part of the anti-leukocyte antibody on the carrier. When the amount of the part of the anti-leukocyte antibody is small to excess, the inducer has little the effect by the anti-leukocyte antibody. On the other hand, when the amount is large to excess, the nonspecific adsorption and cytotoxicity are caused. Therefore the tumor-lysing cell inducer has from 1 x 10⁻⁹ »mol/cm² to 1 x 10⁻² »mol/cm², preferably from 5 x 10⁻⁶ »mol/cm² to 5 x 10⁻⁴ »mol/cm², of the fixed part of the anti-leukocyte antibody on its surface.

The tumor-lysing cell inducer of the invention is one wherein the part of the anti-leukocyte antibody is fixed in the water-insoluble carrier and can have any shape, for instance it is in the shape of particles, a plate, a membrane, and a fiber. When the tumor-lysing cell inducer packed in a column is used, there is preferable an inducer capable of forming gaps through which cells contained in a body fluid can sufficiently pass. For instance, when the inducer is in the shape of the particles, the powdery inducer is not preferable and particles having a particle size of not less than 200 »m are preferable. More detailedly, it is preferable as the inducer to use particles from which particles having a particle size being small to excess and particles having a particle size being large to excess are removed. Further, it is more preferable to use particles having an average particle size of 200 to 1,000 »m and having a narrow particle size distribution. Also, when the inducer is in the shape of a hollow fiber, it is preferable that an inside diameter of the hollow fiber is not less than 5 »m. When the inducer is in the shape of a fiber which is not hollow, it is preferable that an outside diameter is not less than 1 »m.

It is preferable that the surface of the tumor-lysing cell inducer is smooth. When the surface is rough, the nonspecific adsorption is increased and the selective activation is lowered.

The thus obtained tumor-lysing cell inducer by fixing the part of the anti-leukocyte antibody in the solid, the water-insoluble carrier can produce the excellent effect for activating leukocytes, which cannot be expected from soluble antibodies.

The term "tumor-lysing cell" used herein means a cell which is derived from a leukocyte and has a function capable of lysing tumor cells.

In general, a case that cells are stimulated to induce a certain function from the cells is called "the activation of cells", and there are various kinds of the activations. Typical examples of the activations (and its measuring method) are described as below.
(1) The proliferation faster accelerates than usual. (an amount of uptake of ³H-TdR, MTT method, a consumed amount of glucose)
(2) A produced amount of IL-2 is increased. (method according to IL-2-dependent cells)
(3) An amount of IL-2 receptor is increased. (a labeled antibody method using IL-2-dependent cells)
(4) Killer activation (cytotoxicity property against other cells is measured)
The term "tumor-lysing cell induction" used herein means the activation concerned to the killer activation as mentioned above.

Any way can apply to the method for inducing the tumor-lysing cells from the leukocyte in the body fluid, using the tumor-lysing cell inducer of the present invention.

Typical examples of the inducing methods are, for instance, a method in which a body fluid removed from a body is packed in a bag, the tumor-lysing cell inducer is mixed with the body fluid in the bag to induce the tumor-lysing cells, and the tumor-lysing cell inducer is filtered off to obtain the body fluid containing the tumor-lysing cells; and a column method in which a body fluid is passed through the inducers packed in a container having an inlet and an outlet for body fluid, and a filter through which the body fluid can be passed but the tumor-lysing cell inducer is not passed, the filter being attached to the outlet. Both methods can be used. The column method is easy in operation. Further, according to the column method, tumor-lysing cells can be obtained from a body fluid, particularly leukocytes in a blood, of a patient by incorporating the column wherein the inducer is packed into an extracorporeal circulation. The tumor-lysing cell inducers of the present invention are suitable for use of the column method.

There is preferable a method wherein the tumor-lysing cell inducers are packed in the column, the column wherein the inducers are packed is incorporated into an extracorporeal circulation, and tumor-lysing cells are induced according to the on-line information processing system, since according to such a method, a large amount of the body fluid can be treated in a short time.

Next, the device for inducing tumor-lysing cells of the present invention, utilizing the inducer as mentioned above is explained by means of Fig. 1 which is a cross sectional view of one embodiment of the devices for inducing tumor-lysing cells of the present invention.

In Fig. 1, 1 shows an inlet of a body fluid, 2 shows an outlet of the body fluid, 3 shows the tumor-lysing cell inducer of the present invention, 4 and 5 show filters through which the body fluid and ingredients contained in the body fluid can be passed but the tumor-lysing cell inducer cannot be passed, 6 shows a column, and 7 shows a container.

A shape and a kind of a material of the container 7 are not particularly limited. Preferable examples of the containers are, for instance cylindrical containers having a volume of about 150 to 400 mℓ and a diameter of about 4 to 10 cm.

The present invention is more specifically described and explained by means of the following Examples in which all percents and parts are by weight unless otherwise noted.

### Example 1

### Preparation of a part of antibody, [F(ab')₂]

An antibody (IgG) was cut into F(ab')₂ according to a usual manner. That is, 2 mℓ of antibodies (OKT 3, commercially available from Ortho Diagnostic Systems Inc.) with a concentration of 25 »g/mℓ was dialyzed against a 0.1 M sodium acetate buffer solution (pH 4.5), to which 1.0 »g of pepsin (commercially available from Toyoboseki Kabushiki Kaisha) was added. The mixture was allowed to stand at 37°C for 8 hours, then a pH of the resulting mixture was adjusted with a 1 N aqueous solution of sodium hydroxide to 8.0. The resulting mixture was passed through a Sephadex G-150® (commercially available from Pharmacia LKB Biotechnology Inc.) column (0.5 x 15 cm), using a 0.1 M sodium borate buffer solution (pH 8.0) as a developing solvent to separate an F(ab')₂ fraction. The F(ab')₂ fraction was dialyzed against a 0.1 M sodium phosphate buffer solution (pH 6.0).

### Preparation of a tumor-lysing cell inducer

Four grams of CNBr-activated Sepharose® 6MB was swelled with a little amount of a 1 mM aqueous solution of HCℓ for 15 minutes, and it was washed with the aqueous solution of HCℓ (800 mℓ), further a coupling buffer (0.5 M NaCℓ, 0.1 M NaHCO₃, pH 8.3, 20 mℓ).

To a solution wherein the prepared F(ab')₂ was dissolved in the coupling buffer was added the washed gels with a suck dry, and the reaction was conducted at 4°C overnight.

After washing with the coupling buffer, a blocking buffer (0.2 M glycine, 0.5 M NaCℓ, 0.1 M NaHCO₃, pH 8.3, 16 mℓ) was added to the reaction mixture, and the reaction was conducted at room temperature for 2 hours.

The obtained reaction mixture was washed with two kinds of post-treatment buffers total six times, that is, it was washed with a post-treatment buffer (0.5 M NaCℓ, 0.1 M AcNa, pH 4.0) alternately with a post-treatment buffer (0.5 M NaCℓ, 0.1 M Tris, pH 8.0), each washing being conducted three times. Thus, a tumor-lysing cell inducer wherein 5 x 10⁻⁶ »mol/cm² of F(ab')₂ which was a part of anti-leukocyte antibody was fixed in the carrier was obtained.

### Preparation of a leukocyte suspension

A solution wherein leukocytes were suspended was prepared as follows:
A peripheral blood containing heparine was placed on a layer of Ficoll-Paque® (commercially available from Pharmacia LBK Biotechnology Inc.) in the state of a layer, the leukocytes were separated by specific density centrifugation method. The obtained leukocyte fraction was washed with an isotonic solution thoroughly, and a concentration of the washed fraction was adjusted with an isotonic solution having a pH of 7.2 to 4 x 10⁶ cells/mℓ.

### Inducing operation

Into the tumor-lysing cell inducer was pipetted 200 »ℓ/well (8 x 10⁵ cells/mℓ) of the leukocyte suspension, and the stimulation was conducted for 10 minutes. Then, it was transferred to a microplate in which the antibody was not fixed and the culture was conducted for 24 hours.

### Measuring method of inducing effect

Amounts of glucoses consumed and the killer activity were measured as follows:

### (1) Measurement of an amount of glucose consumed

After stimulating, a concentration of the leukocyte suspension was adjusted to 4 x 10⁶ cells/mℓ, which was used as a sample. Also, a concentration of the leukocyte suspension which was not stimilated was adjusted to the same concentration as the sample, which was used as a control. The sample and the control were cultured respectively on a FCS 10 % RPMI 1640 medium in a carbon dioxide incubator for 24 hours. Glucose concentrations in the media before and after the culture were measured (glucose B test Wako, commercially available from Wako Purechemical Industries, Ltd.), and an amount of the glucose consumed was calculated.

### (2) Measurement of killer activity

After stimulating, a concentration of the leukocyte suspension was adjusted to 4 x 10⁶ cells/mℓ, which was used as a sample. Also, a concentration of the leukocyte suspension which was not stimulated was adjusted to the same concentration as the sample, which was used as a control. The sample and the control were cultured respectively on an RPMI 1640 medium in a carbon dioxide incubator for 24 hours. Killer activity against HeLa-S3 cells after culturing was measured according to ⁵¹Cr-release method.

The results are shown in Table 1.

**Table 1**

| | |
|---|---|
| Amount of glucose consumed (control) | 5.5 ± 1.7 % |
| | 7.9 ± 6.2 % |
| Killer activity (against HeLa-S3) (control) | 59.2 ± 4.5 % |
| | 20.5 ± 0.3 % |

## Claims

1. A tumor-lysing cell inducer comprising a part of anti-leukocyte antibody, said part being fixed in the surface of a water-insoluble carrier in an amount of 1 x 10⁻⁹ »mol/cm² to 1 x 10⁻² »mol/cm².

2. The inducer of Claim 1, wherein not less than two kinds of said parts are fixed in said carrier.

3. The inducer of Claim 1, wherein said carrier is hydrophilic.

4. The inducer of Claim 1, wherein said anti-leukocyte antibody is an antibody against at least one surface antigen of the lymphocyte selected from the group consisting of CD2, CD3, CD4, CD8 and CD16.

5. A method for inducing tumor-lysing cells which comprises contacting a body fluid containing leukocytes with a tumor-lysing cell inducer, said tumor-lysing cell inducer comprising a part of anti-leukocyte antibody, said part being fixed in the surface of a water-insoluble carrier in an amount of 1 x 10⁻⁹ »mol/cm² to 1 x 10⁻² »mol/cm².

6. A device for inducing tumor-lysing cells which comprises a tumor-lysing cell inducer comprising a part of anti-leukocyte antibody, said part being fixed in the surface of a water-insoluble carrier in an amount of 1 x 10⁻⁹ »mol/cm² to 1 x 10⁻² »mol/cm², and a container having an inlet and an outlet for body fluid and a preventing means of the outpouring of said inducer from the container, said inducer being packed in said container.

## Patentansprüche

1. Induktor für tumorlysierende Zellen, umfassend einen Teil eines Antileukozytenantikörpers, wobei der Teil in der Oberfläche eines wasserunlöslichen Trägers in einer Menge von 1 x 10⁻⁹ »mol/cm² bis 1 x 10⁻² »mol/cm² fixiert ist.

2. Induktor nach Anspruch 1, wobei nicht weniger als zwei Arten der Teile auf dem Träger fixiert sind.

3. Induktor nach Anspruch 1, wobei der Träger hydrophil ist.

4. Induktor nach Anspruch 1, wobei der Antileukozytenantikörper ein Antikörper gegen wenigstens ein Oberflächenantigen der Lymphozyten, ausgewählt aus der Gruppe bestehend aus CD2, CD3, CD4, CD8 und CD16, ist.

5. Verfahren zur Induktion tumorlysierender Zellen, welches umfaßt den Kontakt einer Körperflüssigkeit, die Leukozyten enthält, mit einem Induktor für tumorlysierende Zellen, wobei der Induktor für tumorlysierende Zellen einen Teil eines Antileukozytenantikörpers umfaßt, wobei der Teil in der Oberfläche eines wasserunlöslichen Trägers in einer Menge von 1 x 10⁻⁹ »mol/cm² bis 1 x 10⁻² »mol/cm² fixiert ist.

6. Vorrichtung zur Induktion tumorlysierender Zellen, welche umfaßt einen Induktor für tumorlysierende Zellen, welcher einen Teil eines Antileukozytenantikörpers umfaßt, wobei der Teil in der Oberfläche eines wasserunlöslichen Trägers in einer Menge von 1 x 10⁻⁹ »mol/cm² bis 1 x 10⁻² »mol/cm² fixiert ist, und einem Behälter mit einem Einlaß und einem Auslaß für Körperflüssigkeiten und Einrichtungen zur Verhinderung des Entweichens des Induktors aus dem Behälter, wobei der Induktor in den Behälter gepackt ist.

## Revendications

1. Inducteur de cellules de lyse de tumeurs comprenant une partie d'un anticorps anti-leucocytes, ladite partie étant fixée à la surface d'un support insoluble dans l'eau en une quantité de 1 x 10⁻⁹ »mole/cm² à 1 x 10⁻² »mole/cm².

2. Inducteur de la revendication 1 dans lequel au moins deux types desdites parties sont fixées dans ledit support.

3. Inducteur de la revendication 1, dans lequel ledit support est hydrophile.

4. Inducteur de la revendication 1, dans lequel ledit anticorps anti-leucocytes est un anticorps contre au moins un antigène de surface du lymphocyte choisi dans le groupe constitué par CD2, CD3, CD4, CD8 et CD16.

5. Procédé pour induire des cellules de lyse de tumeurs dans lequel on met en contact un fluide corporel contenant des leucocytes avec un inducteur de cellules de lyse de tumeurs, ledit inducteur de cellules de lyse de tumeurs comprenant une partie d'anticorps anti-leucocytes, ladite partie étant fixée à la surface d'un support insoluble dans l'eau en une quantité de 1 x 10⁻⁹ »mole/cm² à 1 x 10⁻² »mole/cm².

6. Dispositif pour induire des cellules de lyse de tumeurs qui comprend un inducteur de cellules de lyse de tumeurs, comprenant une partie d'anticorps antileucocytes, ladite partie étant fixée à la surface d'un support insoluble dans l'eau en une quantité de 1 x 10⁻⁹ »mole/cm² à 1 x 10⁻² »mole/cm², et un récipient ayant une entrée et une sortie pour liquide corporel et un moyen pour empêcher le déversement dudit inducteur à partir du récipient, ledit inducteur étant introduit dans ledit récipient.
